# FASCICULE DE BREVET EUROPEEN

(11) **EP 3 419 684 B1**
(45) Date de publication et mention de la délivrance du brevet: **11.12.2019**
(21) Numéro de dépôt: 17710334.8
(22) Date de dépôt: 20.02.2017
(51) Int. Cl.: A61L 31/12, A61L 31/14, A61L 31/16, A61L 15/26, A61L 15/32, A61L 15/44, A61L 15/60, A61L 15/64

(54) **BIOMATÉRIAUX COMPOSITES À LIBÉRATION CONTROLÉE DE PRINCIPE ACTIF, PROCÉDÉ DE PREPARATION ET UTILISATIONS**
ZUSAMMENGESETZTE BIOMATERIALIEN MIT GESTEUERTER FREISETZUNG DES WIRKSTOFFS, HERSTELLUNGSVERFAHREN UND VERWENDUNGEN
COMPOSITE BIOMATERIALS WITH CONTROLLED RELEASE OF THE ACTIVE INGREDIENT, PREPARATION METHOD, AND USES

(30) Priorité: 22.02.2016 FR 1651432
(43) Date de publication de la demande: 02.01.2019
(73) Titulaire: Centre National de la Recherche Scientifique, 75016 Paris (FR); Sorbonne Université, 75006 Paris 6 (FR)
(72) Inventeur: HELARY, Christophe, 94500 Champigny Sur Marne (FR); CORADIN, Thibaud, 75005 Paris (FR)
(74) Mandataire: Goulard, Sophie
(86) Numéro de dépôt international: PCT/FR2017/050377
(87) Numéro de publication internationale: WO 2017/144806

(56) Documents cités:
- Z RUSZCZAK: "Collagen as a carrier for on-site delivery of antibacterial drugs", ADVANCED DRUG DELIVERY REVIEWS, vol. 55, no. 12, 28 novembre 2003 (2003-11-28), pages 1679-1698, XP055125329, ISSN: 0169-409X, DOI: 10.1016/j.addr.2003.08.007 cité dans la demande
- SCHLAPP M ET AL: "COLLAGEN/PLGA MICROPARTICLE COMPOSITES FOR LOCAL CONTROLLED DELIVERY OF GENTAMICIN", JOURNAL OF PHARMACEUTICAL SCIENCES, AMERICAN PHARMACEUTICAL ASSOCIATION, WASHINGTON, US, vol. 92, no. 11, 1 novembre 2003 (2003-11-01), pages 2145-2151, XP001177679, ISSN: 0022-3549, DOI: 10.1002/JPS.10460

## Description

L'invention concerne un biomatériau composite à base de collagène, d'au moins un polymère organique hydrophobe et d'au moins un principe actif, son procédé de préparation, un pansement comprenant un tel biomatériau composite, un renfort de paroi abdominale comprenant un tel biomatériau composite, ainsi que les utilisations dudit biomatériau composite, notamment dans le domaine thérapeutique.

L'invention s'applique en particulier au domaine des biomatériaux qui permettent de délivrer des principes actifs, notamment pour soigner des blessures chroniques.

La peau qui recouvre intégralement le corps humain assure une fonction de barrière protectrice des organes et des tissus face aux agressions extérieures. Son rôle est donc fondamental pour le bon fonctionnement de l'organisme. La peau est composée de trois couches : l'épiderme, le derme et l'hypoderme ou couche sous-cutanée. Après une blessure, la réparation tissulaire ou la cicatrisation se met immédiatement en place. Elle se compose de quatre phases : l'hémostase, la phase inflammatoire, la phase de prolifération et la phase de remodelage. Une blessure chronique est définie comme une plaie de peau qui ne se referme pas au-delà de 42 jours. Ces plaies peuvent prendre la forme d'ulcères de jambes, d'ulcères de pied chez le patient diabétique ou d'escarre. Ces plaies chroniques résultent de complications chez des patients atteints de diabète, d'insuffisance vasculaire ou encore de déficiences nutritionnelles. La prévalence de lésions au pied chez le diabétique est de 20% environ chaque année. Ce problème touche chaque année plusieurs millions de personnes dans le monde, le plus souvent âgés de plus de 65 ans et constitue donc un enjeu de taille pour la recherche en médecine régénérative. La différence majeure avec les blessures dites aigües, est que le processus de cicatrisation n'est pas complet et reste bloqué à la phase inflammatoire. Les traitements de blessures chroniques doivent à la fois stopper l'inflammation et promouvoir la régénération tissulaire afin d'obtenir la fermeture de la plaie ainsi que sa cicatrisation.

La technique couramment utilisée pour soigner les plaies chroniques est le débridement de la plaie suivie de sa compression. Il s'agit d'ôter le tissu nécrotique et les adhérences fibreuses qui se sont développées entre les tissus afin de pouvoir nettoyer la plaie. Dans certains cas, cette technique est inefficace, il est alors nécessaire de couvrir la plaie avec un pansement à base de biomatériaux tels que des hydrocolloïdes ou des hydrogels. Le pansement a pour fonction de protéger la plaie d'éventuelles infections, tout en permettant sa fermeture. Les hydrocolloïdes sont définis dans le rapport d'évaluation de la Haute Autorité de Santé comme des pansements constitués de polymères absorbants, dont les propriétés physicochimiques sont liées à la présence de carboxyméthylcellulose. Toutefois, ce type de pansement peut induire un risque de surinfection lié au micro-environnement excessivement humide qu'il génère. Les hydrogels sont des gels physiques ou chimiques composés de chaînes de polymères gonflées par une grande quantité d'eau (environ 70 % du volume total de l'hydrogel). Il est connu d'utiliser des hydrogels à base d'alginate ou de collagène. Toutefois, l'alginate se dégrade assez rapidement au contact des enzymes du corps humain. Quant au collagène, il présente des propriétés mécaniques intrinsèques limitées qui peuvent être améliorées par réticulation à l'aide d'agents chimiques (aldéhydes, carbodiimides, etc...). Toutefois, la toxicité de ces agents chimiques pose problème, ceux-ci pouvant entraîner des effets secondaires indésirables.

Les pansements peuvent être utilisés en association avec un traitement oral ou une composition topique à base d'au moins un principe actif de type antibiotique, anti-inflammatoire ou d'une molécule favorisant la cicatrisation. Cependant, le traitement oral peut se révéler inefficace de par l'accès limité du principe actif à l'épiderme ; et la composition topique requiert généralement des applications cutanées fréquentes et le contact direct entre la blessure et l'air, augmentant le risque d'infection.

Ainsi, les solutions proposées ci-dessus sont soit inefficaces et le traitement de la plaie nécessite alors l'utilisation d'un substitut cutané qui est très coûteux, soit elles n'agissent pas sur toutes les étapes de la cicatrisation et requièrent de combiner plusieurs de ces solutions.

Une autre solution est la libération prolongée d'un principe actif à l'aide d'un support adapté pour permettre l'absorption régulière et soutenue dans le temps dudit principe actif. La libération se doit d'être contrôlée, afin que le médicament soit le plus efficace possible, avec le moins d'effets secondaires et une durée d'action prolongée. Il est d'ailleurs décrit des hydrogels de collagène pur comme support pour la libération contrôlée d'un principe actif. Toutefois, les hydrogels de collagène pur ne retiennent pas suffisamment le principe actif et présentent une tenue mécanique insuffisante [Wallace et al., Advanced Drug Delivery Reviews, 2003, 55, 1631-1649].

Par ailleurs, des matériaux composites collagène-polymère organique ont été proposés pour la libération prolongée de médicaments. En particulier, Ruszczak et al. [Advanced Drug Delivery Reviews, 2003, 55, 1679-1698] ont décrit un matériau composite à base d'une éponge collagène, de microparticules d'acide poly(lactique-co-glycolique) (PLGA) et d'un antibiotique comme principe actif. Le matériau composite sous la forme d'une éponge composite de collagène a été préparé de la façon suivante : des microparticules de PLGA comprenant l'antibiotique ont été préparées par la technique de double émulsion. Les microparticules obtenues ont ensuite été mélangées avec une solution aqueuse de collagène et d'antibiotique, puis la dispersion résultante a été lyophilisée. Dans Ruszczak *et al*., le collagène insoluble (IC) qui sert de constituant de base est dissous dans l'acide acétique à pH 4,5. A ce pH, le collagène initialement fibrillaire se dissout dans l'acide sous forme de triples hélices de collagène et perd sa forme fibrillaire. Comme le procédé de fabrication repose sur la lyophilisation de la solution sans repasser par un pH neutre, le matériau final n'est formé que de triples hélices de collagène. Par ailleurs, il est connu que le collagène des éponges de collagène ne présente pas une structure fibrillaire et striée, comme le rapporte par exemple Vigier et al., Journal of Biomedical Materials Research, 2010, 94A, 2, 556-567. De plus, les éponges de collagène sont connues pour se dénaturer en gélatine à 37°C si elles ne sont pas réticulées par des agents chimiques. Il en résulte que le matériau composite de Ruszczak *et al.* ne présente pas des propriétés de libération contrôlée et prolongée du principe actif (sans une libération initiale rapide) et des propriétés mécaniques optimisées.

D'autres matériaux composites collagène-polymère organique connus sont sous la forme de fibres dudit polymère organique recouvertes d'une fine couche de collagène.

Cependant, les matériaux composites de l'art antérieur ne présentent pas un profil de libération du principe actif optimisé (e.g. libération trop rapide et/ou non linéaire). Par ailleurs, ils n'ont pas des propriétés mécaniques adaptées, notamment une élasticité, une élongation à la rupture et/ou une résistance à la rupture adaptée(s), pour pouvoir être utilisés dans des pansements thérapeutiques (e.g. matériaux extrêmement rigides ou trop lâches et/ou trop fragiles).

Le but de la présente invention est de pallier les inconvénients de l'art antérieur et de fournir un matériau biocompatible, biodégradable, qui présente des propriétés mécaniques adaptées pour pouvoir être utilisé dans le domaine des pansements thérapeutiques, qui puisse favoriser plusieurs étapes du processus de cicatrisation, notamment dans le cas de blessures chroniques, et qui permette de libérer de façon contrôlée au moins un principe actif participant audit processus de cicatrisation ou à la réparation tissulaire.

L'invention a donc pour objet un biomatériau composite synthétique comprenant du collagène, au moins un polymère organique et au moins un principe actif, caractérisé en ce que :
- le polymère organique est biodégradable, biocompatible, hydrophobe et il présente une température de transition vitreuse inférieure ou égale à 50°C, et de préférence inférieure ou égale à 45°C environ, et une masse molaire moyenne allant de 5 à 120 KDa environ,
- le collagène est sous la forme de fibrilles striées dans lesquelles la périodicité des striations est de 67 nm,
- le rapport massique collagène/polymère organique va de 10/1 à 1/3 environ, de préférence de 5/1 à 1/2 environ, et de préférence encore de 2/1 à 2/3 environ,
- le principe actif est un principe actif hydrophobe choisi parmi les anti-inflammatoires, les antibiotiques, les composés favorisant la réparation tissulaire ou la cicatrisation et un de leur mélange.

Le biomatériau composite synthétique de l'invention est biocompatible, biodégradable et il présente des propriétés mécaniques comparables ou améliorées par rapport à un hydrogel de collagène pur, tout en permettant le relargage contrôlé et prolongé d'un ou plusieurs principe actif, notamment sans une libération initiale rapide.

En particulier, le biomatériau composite de l'invention peut libérer de façon quasi-constante un principe actif sur une période d'au moins 7 jours, et de préférence sur une période d'au moins 3 semaines, voire 1 mois.

Le collagène du biomatériau composite est sous la forme de fibrilles striées et le rapport massique collagène/polymère organique va de 10/1 à 1/3 environ, de préférence de 5/1 à 1/2 environ, et de préférence encore de 2/1 à 2/3 environ. Grâce à ces caractéristiques, le biomatériau est homogène et présente de bonnes propriétés mécaniques. En particulier, la structure fibrillaire striée est stable jusqu'à au moins 50°C et ne se dénature pas à la température du corps humain (i.e. 37°C environ). Par ailleurs, le biomatériau peut relarger de façon contrôlée, prolongée et quasi-constante un ou plusieurs principes actifs, notamment en évitant le phénomène de libération initiale rapide du principe actif (également bien connu sous l'anglicisme « *burst release* »).

Le biomatériau composite de l'invention est de préférence exempt de tout solvant organique.

Le biomatériau composite de l'invention peut être sous la forme d'un hydrogel composite ou sous la forme d'un matériau composite sec.

Lorsque le biomatériau composite est sous la forme d'un matériau composite sec, il comprend au plus 10% en masse environ d'eau, et de préférence au plus 5% en masse environ, par rapport à la masse totale du biomatériau composite. Selon une forme de réalisation préférée de l'invention, le matériau composite sec ne comprend pas d'eau de solvatation.

Lorsque le biomatériau composite est sous la forme d'un hydrogel composite, il comprend de 70 à 95% en masse environ d'eau, et de préférence de 80 à 90% en masse environ d'eau, par rapport à la masse totale du biomatériau composite.

Le biomatériau composite de l'invention est de préférence constitué du ou des principes actifs, du ou des polymères organiques, du collagène et éventuellement d'eau ou d'une solution aqueuse de pH allant de 7 à 8 environ (e.g. tampon phosphate salin).

Dans la présente invention, l'expression « polymère organique biodégradable » signifie un polymère organique qui peut être dégradé ou digéré par des microorganismes (e.g. bactéries, champignons, algues), notamment par l'action d'enzymes. Les réactions intervenant lors de la biodégradation chez l'homme sont des réactions d'hydrolyse, c'est-à-dire de coupures de liaisons covalentes par réaction avec l'eau (cf. norme actuelle NFEN 13 432).

Dans la présente invention, l'expression « polymère organique biocompatible » signifie un polymère organique ayant la capacité à ne pas interférer et à ne pas dégrader le milieu biologique dans lequel ils sont utilisés. En particulier, ils ne doivent pas provoquer de réaction inflammatoire forte (e.g. allergies) et/ou ne doivent pas être toxiques pour l'être humain.

Dans la présente invention, l'expression « polymère organique hydrophobe » signifie un polymère organique pour lequel on mesure un angle de contact compris entre 60° et 150° environ, de préférence entre 60 et 110° environ, et plus préférentiellement encore entre 65 et 90° environ, par exemple selon la méthode de mesure Wihelmy détaillée dans les exemples ci-après.

Ainsi, les polymères organiques conformes à l'invention sont insolubles dans les fluides biologiques.

La présence d'au moins un polymère organique hydrophobe dans le biomatériau composite de l'invention permet l'encapsulation d'un ou plusieurs principes actifs et leur libération contrôlée.

Le polymère organique a de préférence une température de transition vitreuse inférieure ou égale à 40°C environ, et de préférence encore inférieure ou égale à 38°C environ.

Par ailleurs, il est avantageux que le polymère organique ait une température de transition vitreuse lorsqu'il est associé au collagène, qui soit inférieure ou égale à 37°C environ, de préférence inférieure ou égale à 32°C environ, et de préférence encore inférieure ou égale à 28°C environ.

En effet, une température de 28°C environ correspond à la température d'équilibre d'un pansement sur la peau (température à l'interface air ambiant-température physiologique). Une température de transition vitreuse du polymère organique lorsqu'il est associé au collagène inférieure ou égale à 40°C environ permet d'obtenir un biomatériau composite présentant des propriétés mécaniques comparables, voire améliorées par rapport à du collagène pur (élasticité, élongation à la rupture et/ou résistance à la rupture).

Le polymère organique peut être choisi parmi les polyesters aliphatiques, les polyéthylènes glycols, les polyanhydrides et les poly(ortho-esters).

De préférence, le polymère organique est un polyester aliphatique, notamment choisi parmi un polyglycolide [i.e. poly(acide glycolique) ou PGA], un polylactide [i.e. poly(acide lactique ou PLA], un copolymère de glycolide et de lactide (PLGA), un polylactone (e.g poly(ε-caprolactone)), et un polyhydroxyalcanoate (e.g. polyhydroxyvalérate, poly(hydroxybutyrate)).

Selon une forme de réalisation particulièrement préférée de l'invention, le polymère organique est un copolymère de glycolide et de lactide.

Le copolymère de glycolide et de lactide peut présenter un rapport molaire lactide/glycolide allant de 50/50 à 85/15 environ, de préférence allant de 50/50 à 65/35 environ.

Le polymère organique peut présenter une masse molaire moyenne allant de 5 KDa à 60 KDa environ, et de préférence encore de 7 KDa à 20 KDa environ.

La teneur en polymère organique du biomatériau composite lorsque celui-ci est sous la forme d'un hydrogel composite est d'au moins 10 mg/ml environ, de préférence va de 20 à 100 mg/ml environ, et de préférence encore va de 30 à 60 mg/ml environ.

Le polymère organique du biomatériau composite de l'invention est sous la forme de nanodomaines, notamment présentant une taille moyenne inférieure ou égale à 700 nm environ, de préférence inférieure ou égale à 600 nm environ, et de préférence encore allant de 100 à 500 nm environ.

La taille moyenne de ces nanodomaines est déterminée par microscopie électronique en transmission (MET).

En d'autres termes, le polymère organique n'est pas sous la forme de fibres ou de microparticules comme c'est le cas dans les matériaux composites de l'art antérieur.

Le collagène est de préférence un collagène de type I ou III, et de préférence encore un collagène de type I. Le collagène fibrillaire de type I est une protéine naturelle présente à 70% en masse environ dans la matrice extracellulaire de la peau, il contribue à la structure mécanique des tissus conjonctifs et est le substrat pour l'adhérence des cellules.

La teneur en collagène du biomatériau composite lorsque celui-ci est sous la forme d'un hydrogel composite est d'au moins 10 mg/ml environ, de préférence de 20 à 100 mg/ml environ, et de préférence encore de 30 à 60 mg/ml environ.

Une teneur minimum de collagène d'au moins 10 mg/ml environ dans le biomatériau composite permet d'éviter sa dégradation rapide dans le milieu physiologique lors d'une application cutanée (e.g. sous la forme d'un pansement).

Dans le matériau de l'invention, les dites fibrilles ne sont généralement pas ordonnées. Le matériau comprend donc majoritairement, voire uniquement, des domaines isotropiques.

Le principe actif de type antibiotique peut être de la gentamycine, de la ryfamicine ou de l'amoxicilline.

Le principe actif de type anti-inflammatoire peut être de la dexaméthasone ou un antalgique tel que l'ibuprofène.

Le principe actif favorisant la cicatrisation ou la réparation tissulaire peut être la spironolactone.

La spironolactone est connue pour être un antagoniste du récepteur minéralocorticoïde et pour favoriser la réparation des plaies cutanées.

Selon une forme de réalisation particulièrement préférée de l'invention, le principe actif est la spironolactone.

Dans la présente invention, l'expression « principe actif hydrophobe » signifie un principe actif ayant un coefficient de partage P dans un système octanol/eau tel que log P > 0, de préférence log P > 2, et de préférence encore log P > 3.

Le coefficient de partage P est bien connu de l'homme du métier. Il est égal au rapport des concentrations d'un soluté (principe actif) dans deux phases : P = C'/C, C' correspondant à la concentration du soluté dans un solvant organique saturé d'eau, et C correspondant à la concentration du soluté dans l'eau saturé de solvant organique.

La capacité de dissolution dans l'eau d'un principe actif hydrophobe tel que celui utilisé dans le biomatériau composite de l'invention est généralement inférieure à 50 mg/l environ.

La teneur du biomatériau composite en principe actif dépend du principe actif que l'on souhaite incorporer et donc de l'application visée.

En particulier, le biomatériau composite sous la forme d'un hydrogel composite peut comprendre de 0,1 à 10 mg/ml environ de spironolactone.

Le biomatériau composite de l'invention sous la forme d'un hydrogel composite peut présenter une élasticité (i.e. un module de Young) d'au moins 10000 Pa environ, de préférence d'au moins 40000 Pa environ, et de préférence encore allant de 50000 à 100000 Pa environ.

Le biomatériau composite de l'invention sous la forme d'un hydrogel composite peut présenter un module de cisaillement d'au moins 3000 Pa environ, de préférence d'au moins 5000 Pa environ, et de préférence encore allant de 10000 à 60000 Pa environ, à une fréquence comprise entre 1 et 10 Hz.

Le biomatériau composite de l'invention peut présenter une élongation à la rupture d'au moins 10% environ, de préférence d'au moins 30% environ, et de préférence encore allant de 40 à 75% environ.

Le biomatériau composite de l'invention peut présenter une résistance à la rupture d'au moins 2,5 MPa environ, de préférence d'au moins 3 MPa environ, et de préférence encore allant de 3,5 à 12 MPa environ.

Le biomatériau composite de l'invention est sous la forme d'une matrice de fibrilles striées de collagène dans laquelle des nanodomaines constitués dudit polymère organique sont dispersés de façon homogène (réseau tridimensionnel homogène). En particulier, les nanodomaines dudit polymère organique présentent une taille moyenne inférieure ou égale à 700 nm environ, de préférence inférieure ou égale à 600 nm environ et de préférence encore allant de 100 à 500 nm environ.

L'invention a également pour objet un procédé de préparation d'un biomatériau composite tel que défini dans le premier objet de l'invention, caractérisé en ce qu'il comprend au moins les étapes suivantes :
i) la préparation d'un hydrogel de collagène sous la forme de fibrilles striées dans lesquelles la périodicité des striations est de 67 nm, la concentration en collagène dans l'hydrogel étant d'au moins 10 mg/ml environ,
ii) la déshydratation de l'hydrogel tel que préparé à l'étape i) par incubation dudit hydrogel dans plusieurs solutions mixtes successives [solvant organique/solvant aqueux] présentant une proportion croissante en solvant organique, les dits solvants aqueux et organiques étant miscibles, suivi d'une dernière incubation dans une solution pure dudit solvant organique,
iii) la mise en contact de l'hydrogel déshydraté de l'étape ii) avec une solution d'imprégnation comprenant au moins un polymère organique et au moins un principe actif tels que définis dans le premier objet de l'invention, le rapport volumique : volume de la solution d'imprégnation/volume de l'hydrogel déshydraté étant supérieur ou égal à 3 environ,
iv) le rinçage de l'hydrogel imprégné de l'étape iii) avec un solvant organique, puis avec un solvant aqueux.

Ainsi, dans le procédé de l'invention, la structure fibrillaire et striée du collagène est conservée. Le procédé est simple, économique et il permet d'obtenir un matériau biocompatible, biodégradable, qui présente des propriétés mécaniques adaptées pour pouvoir être utilisé dans le domaine des pansements thérapeutiques, qui puisse favoriser plusieurs étapes du processus de cicatrisation, notamment dans le cas de blessures chroniques, et qui permette de libérer de façon contrôlée au moins un principe actif participant audit processus de cicatrisation ou à la réparation tissulaire.

De préférence, la concentration en collagène de l'hydrogel issu de l'étape i) va de 20 à 100 mg/ml environ, et de préférence encore de 30 à 60 mg/ml environ. Une concentration de 30 à 60 mg/ml environ est particulièrement adaptée pour obtenir un biomatériau composite ayant une capacité d'hydratation et une capacité de gonflement élevées, une bonne élasticité et une bonne stabilité contre la dégradation enzymatique.

L'étape i) peut être effectuée selon les sous-étapes suivantes :
i-1) la préparation d'une solution de collagène acido-soluble dont la teneur en collagène varie de 1 à 5 mg/ml environ, et est de préférence de l'ordre de 5 mg/ml,
i-2) l'évaporation à l'air de la solution de l'étape i-1), et
i-3) la mise en contact de la solution de l'étape i-2) avec une base.

L'étape i-1) peut être effectuée par tout moyen connu de l'homme du métier, notamment par extraction de collagène dans une espèce bovine, murine ou porcine.

Une solution acido-soluble est une solution aqueuse acide dans laquelle le collagène peut être dissous. Son pH est généralement inférieur à 4 environ, de préférence inférieur à 3 environ, en présence d'acides, de préférence d'acide acétique par exemple à 0,5 M environ.

L'étape i-2) est progressive et permet la concentration du collagène au sein de la solution. Elle peut durer de 3 jours à 2 semaines environ. A l'issue de la sous-étape i-2), la concentration de la solution en collagène est d'au moins 10 mg/ml environ, de préférence de 20 à 100 mg/ml environ, et de préférence encore de 30 à 60 mg/ml environ (i.e. le collagène a la concentration souhaitée pour former le biomatériau composite de l'invention).

L'étape i-3) permet de soumettre la solution de collagène concentrée à une augmentation de pH qui induit la gélification du collagène et sa fibrillogénèse.

Elle est effectuée avantageusement par mise en contact de la solution de l'étape i-2) avec une atmosphère gazeuse basique, en particulier une atmosphère de NH₃ ou de (NH₄)₂CO₃.

L'étape i-3) dure généralement entre 12h et 24h environ.

L'étape i) peut comprendre en outre une sous-étape i-4) de rinçage de l'hydrogel de l'étape i-3), notamment avec plusieurs solutions de tampon phosphate salin (PBS), afin d'éliminer la base.

L'étape i) peut comprendre en outre une sous-étape i-2') entre les sous-étapes i-2) et i-3) au cours de laquelle la solution de l'étape i-2) est transvasée dans un moule et centrifugée. Cette étape i-2') permet d'aplanir les irrégularités surfaciques de la solution concentrée (et visqueuse) de collagène de l'étape i-2).

L'étape i) peut également être effectuée selon la procédure telle que décrite dans Wang et al., Nature Materials, 2012, 11, 724-733.

L'étape ii) permet de conduire à un matériau déshydraté (i.e. sans eau) de collagène pur.

Dans un mode de réalisation particulier, le solvant organique est choisi parmi le tétrahydrofurane (THF) et le diméthylsulfoxyde (DMSO).

Dans un mode de réalisation particulier, le solvant aqueux est choisi parmi l'eau et un tampon phosphate salin (PBS).

L'étape ii) peut être conduite en incubant l'hydrogel de l'étape i) dans une solution mixte solvant organique/solvant aqueux dont la teneur en solvant organique est de 20 à 30% en volume environ, puis dans une solution mixte solvant organique/solvant aqueux dont la teneur en solvant organique est de 40 à 50% en volume environ, puis dans une solution mixte solvant organique/solvant aqueux dont la teneur en solvant organique est de 60 à 70% en volume environ, puis dans une solution mixte solvant organique/solvant aqueux dont la teneur en solvant organique est de 80 à 95% en volume environ, puis dans une solution pure dudit solvant organique.

L'incubation dans chacune des solutions peut durer de 30 min à 2h environ, et de préférence de 45 min à 1h15 environ, et de préférence encore de l'ordre de 1h environ.

L'étape ii) permet de créer et/ou de conserver une porosité homogène au sein du collagène. Cette étape ii) est essentielle dans le procédé de l'invention puisqu'elle permet d'une part d'avoir des résultats reproductibles, et d'autre part d'obtenir une bonne imprégnation de l'hydrogel lors de l'étape suivante iii). On obtient ainsi un biomatériau composite homogène (e.g. absence de domaines microscopiques de polymère organique dans le biomatériau composite et conservation de la structure fibrillaire striée du collagène) qui présente de bonnes propriétés de libération prolongée du principe actif tout en garantissant de bonnes propriétés mécaniques.

L'étape iii) permet d'imprégner l'hydrogel de l'étape ii) avec une solution de polymère organique et de principe actif.

Grâce à la porosité de l'hydrogel et à un milieu propice à la diffusion du polymère organique, une quantité importante de polymère organique peut être incorporée dans l'hydrogel.

L'étape iii) est de préférence effectuée en préparant une solution d'imprégnation comprenant le principe actif et le polymère organique, puis en incubant l'hydrogel déshydraté de collagène pur de l'étape ii) dans la solution d'imprégnation.

En particulier, la solution d'imprégnation est préparée en dissolvant le principe actif à la concentration souhaitée dans un solvant organique, puis en ajoutant à la solution précédente le polymère organique à la concentration souhaitée, et enfin en le dissolvant dans ladite solution précédente.

L'étape iii) est de préférence effectuée à température ambiante.

Le solvant organique peut être choisi parmi tous les solvants qui permettent de solubiliser à la fois le principe actif et le polymère organique. À titre d'exemple, le solvant organique peut être du THF ou du DMSO.

La concentration en polymère organique de la solution d'imprégnation peut aller de 20 à 500 mg/ml environ, et de préférence de 80 à 160 mg/ml environ.

La concentration en principe actif de la solution d'imprégnation peut varier de 0,1 à 100 mg/ml environ, de préférence de 0,5 à 40 mg/ml environ, et de préférence encore de 1 à 10 mg/ml environ.

Le rapport volumique : volume de la solution d'imprégnation/volume de l'hydrogel déshydraté varie de préférence de 3/1 à 20/1 environ, et est de préférence de l'ordre de 5/1.

L'étape iii) dure généralement de 12h à 24h environ.

Le solvant organique de l'étape iv) peut être du THF ou du DMSO.

Le solvant aqueux de l'étape iv) peut être un tampon phosphate salin (PBS), un fluide simulant les fluides corporels (bien connu sous l'anglicisme « *Sérum body Fluid* » ou SBF), de l'eau ou un milieu de culture cellulaire.

L'étape iv) est de préférence conduite en rinçant l'hydrogel imprégné de l'étape iii) dans plusieurs bains de solvant organique, notamment pendant 15 secondes à 1 minute environ, et de préférence pendant 30 secondes environ ; et en rinçant l'hydrogel imprégné de l'étape iii) dans plusieurs bains de solvant aqueux, notamment pendant 15 minutes à 1 heure environ, et de préférence pendant 30 minutes environ.

Le rinçage avec un solvant organique permet d'éliminer l'excès de polymère qui n'a pas été absorbé par l'hydrogel de collagène.

Le rinçage avec un solvant aqueux permet de figer le polymère au sein de l'hydrogel de collagène. Sans cette dernière étape de rinçage avec un solvant aqueux, le biomatériau composite ne présente pas une tenue mécanique suffisante et il est fragile.

A l'issue de l'étape iv), le biomatériau composite est sous la forme d'un hydrogel composite.

Le procédé peut comprendre en outre une étape v) de lyophilisation du biomatériau composite de l'étape iv).

A l'issue de l'étape v), le biomatériau composite est sous la forme d'un matériau composite sec. Le matériau composite sec est de préférence exempt de tout solvant organique ou aqueux.

Le matériau composite sec peut alors être conservé à long terme.

Le procédé peut comprendre en outre une étape vi) d'hydratation du matériau composite sec. Cette étape vi) permet de reformer le matériau composite sous la forme d'un hydrogel composite.

L'étape vi) peut être effectuée en présence d'une solution aqueuse choisie parmi l'eau, un tampon phosphate salin, un fluide simulant les fluides corporels et un milieu de culture cellulaire.

Les étapes v et vi) permettent d'éliminer complètement tout solvant organique qui aurait pu être encore éventuellement présent dans l'hydrogel composite obtenu à l'issue de l'étape de rinçage iv).

L'invention a pour troisième objet un biomatériau composite conforme au premier objet de l'invention, pour son usage médical.

L'invention a pour quatrième objet un biomatériau composite conforme au premier objet de l'invention, pour son utilisation dans le traitement de plaies chroniques. Ces plaies chroniques sont généralement rencontrées dans les ulcères de pied, les ulcères veineux ou les escarres.

Dans un mode de réalisation particulier, le biomatériau composite conforme à l'invention est laissé au contact de la peau pendant au moins une semaine.

L'invention a pour cinquième objet un biomatériau composite conforme au premier objet de l'invention, pour son utilisation dans le traitement préventif des infections après une chirurgie cardiaque ou colorectale.

L'invention a pour sixième objet un pansement thérapeutique comprenant une couche interne et une couche externe constituée d'un pansement secondaire choisi parmi un adhésif, une compresse, un bandage et un de leurs mélanges, caractérisé en ce que la couche interne comprend un biomatériau composite conforme au premier objet de l'invention.

Le pansement secondaire peut en particulier être un pansement poreux, de la gaze stérile, un film ou une mousse polyuréthane, ou un film non tissé de polyamide/polyester.

La couche interne est en contact avec la peau et la couche externe maintient la couche interne.

Le pansement peut libérer le principe actif, et en particulier la spironolactone, à dose constante pendant au moins une semaine.

L'invention a pour septième objet un biomatériau composite conforme au premier objet de l'invention, pour son utilisation dans le traitement des hernies de la paroi abdominale ou des éventrations.

L'invention a pour huitième objet un renfort de paroi abdominale comprenant un biomatériau composite conforme au premier objet de l'invention.

### Exemples

Les matières premières utilisées dans les exemples, sont listées ci-après :
- PLGA 7-17 KDa, 719897-5G, Sigma, 50:50 (Lactide:Glycolide),
- PLGA 24-38 KDa, 719870-5G, Sigma, 50:50 (Lactide:Glycolide),
- PLGA 30-60 KDa, P2191-5G, Sigma, 50:50 (Lactide:Glycolide),
- PLA 10 KDa, 764620-5G, Sigma
- PLA 30 KDa, 767344-5G, Sigma
- PCL 14 KDa, 440752-5G, Sigma,
- tendons de queues de rat, Gibco A1048301,
- spironolactone, S3378-1G, Sigma,
- dexaméthasone, D4902-1G, Sigma, et
- aldostérone, A9477-25MG, Sigma.

Sauf indications contraires, tous les matériaux ont été utilisés tels que reçus des fabricants.

Les matériaux préparés ou commerciaux ont été caractérisés par microscopie électronique à transmission (MET, dosage de l'hydroxyproline, mesure de l'angle de contact, mesure de l'élongation à la rupture, mesure de la résistance à la rupture, mesure de l'élasticité, mesure de la toxicité, mesure du suivi de libération du principe actif par spectroscopie UV et mesure de l'activité biologique de la spironolactone.

L'analyse par microscopie électronique à transmission (MET) a été effectuée à l'aide d'un appareil vendu sous la dénomination commerciale Technai Spirit G2, par la société FEI.

La mesure de l'angle de contact a été réalisée à l'aide d'un tensiomètre vendu sous la dénomination commerciale DSA 30 par la société KRUSS. Cette mesure a été effectuée selon la méthode Wihelmy suivante : on dépose sur une lame de verre propre et sèche une solution de polymère organique de concentration 160 mg/ml environ dissous dans du tétrahydrofurane (THF). On sèche pour former un film du polymère organique à la surface de la lame de verre. Puis, on dépose une goutte d'eau sur la lame de verre recouverte de polymère organique. Cette opération est effectuée à 25°C environ. Le tensiomètre mesure la tension de surface entre l'eau et la lame de verre recouverte de polymère organique, et calcule l'angle de contact avec l'eau qui en résulte. Plus l'angle de contact est important, plus le polymère organique est hydrophobe.

L'élasticité a été déterminée à 25°C à l'aide d'un appareil vendu sous la dénomination commerciale Electroforce 3220 par la société Bose.

Le module de cisaillement a été déterminé à 25°C à l'aide d'un appareil vendu sous la dénomination commerciale MCR 301 par la société Anton Paar.

L'élongation à la rupture a été déterminée à 25°C à l'aide d'un appareil vendu sous la dénomination commerciale Electroforce 3220 par la société Bose.

La résistance à la rupture a été déterminée à 25°C à l'aide d'un appareil vendu sous la dénomination commerciale Electroforce 3220 par la société Bose.

Le dosage de l'hydroxyproline permet de déterminer la concentration de collagène dans les différentes solutions ou matériaux. L'hydroxyproline est un acide aminé très présent dans la chaîne polypeptidique du collagène. Le dosage a été réalisé de la façon suivante : la solution de collagène à tester a été hydrolysée en conditions acides (HCl 6 M) à 108°C environ. L'hydroxyproline a ainsi été libérée, puis le mélange résultant a été séché. L'hydroxyproline a été oxydée par de la Chloramine T puis complexée avec du diméthylamino-4-benzaldéhyde (DMBA) pour donner un produit coloré. La détermination de la concentration s'est faite par mesure spectrophotométrique à 557 nm par comparaison avec une gamme étalon.

La mesure de la toxicité des matériaux a été réalisée de la façon suivante : des fibroblastes dermiques primaires humains ont été ensemencés sur plaque de culture. Les hydrogels composites ont ensuite été incubés avec les fibroblastes pendant 1 ou 6 jours. La viabilité cellulaire a été mesurée par un test métabolique (AlamarBlue Assay®) et comparée avec celle de fibroblastes contrôles (sans ajout d'hydrogel composite).

La mesure du suivi de libération du principe actif a été effectuée par spectroscopie UV à l'aide d'un appareil vendu sous la dénomination commerciale Uvikon XL, par la société NorthStar Scientific.

La mesure de l'activité biologique de la spironolactone a été réalisée de la façon suivante : les cellules H9C2 sont des myoblastes génétiquement modifiés par l'incorporation du gène rapporteur codant pour la luciférase en aval du promoteur possédant les séquences liant le complexe : aldostérone avec son récepteur. Lorsque l'aldostérone a été ajoutée, la luciférase a été exprimée et détectée par bioluminescence. Lorsque la spironolactone libérée par les hydrogels testés est active, son incubation avec les H9C2 inhibe l'aldostérone et rend le signal de bioluminescence nul. Cette inhibition rend compte de l'activité biologique de la spironolactone sur des H9C2 incubées avec de l'aldostérone. L'activité de la spironolactone a été mesurée directement sur le liquide de relargage de la spironolactone provenant des différents types d'hydrogels testés.

### Exemple 1 : préparation d'un biomatériau composite synthétique conforme au premier objet de l'invention

### 1.1 Extraction du collagène

Le collagène a été extrait de tendons de queues de rats, connus pour être très riches en collagène de type I. Pour ce faire, des tendons de rats ont été rincés avec du tampon phosphate salin (PBS) en les centrifugeant pendant 5 min environ, à 4°C environ et à 3000 G (G est l'unité de mesure de la vitesse de centrifugation et correspond à l'accélération de la pesanteur) jusqu'à ce que la solution devienne limpide, exempte de cellules et de sang. Ils ont ensuite été rincés avec une solution de NaCl 4 M environ, afin de détruire toutes les cellules restantes. Après un nouveau rinçage au PBS pour éliminer toute trace de NaCl, les tendons lavés ont été mélangés avec une solution d'acide acétique 0,5 M environ pendant 24h environ, puis le mélange résultant a été centrifugé à 3000 G environ pendant 20 min environ. La solution résultante comprenait alors des triples hélices de collagène I en présence d'autres protéines. Le collagène I a été précipité sélectivement par ajout goutte à goutte d'une solution de NaCl 4 M environ à la solution résultante pour obtenir une concentration en NaCl finale de 0,7M. Le mélange résultant a ensuite été centrifugé à 3000 G et le précipité obtenu a été solubilisé dans de l'acide acétique 0,5 M pour former une solution comprenant essentiellement du collagène I. Cette solution a été dialysée contre le même solvant afin d'éliminer totalement le NaCl (4 bains de 24 h environ) et centrifugée à 21 000 G environ pendant 3h environ pour éliminer les derniers agrégats colloïdaux.

La solution de collagène ainsi préparée a été conservée à 4°C environ, afin de préserver sa structure en triple hélice. Le dosage de l'hydroxyproline dans cette solution a permis de déterminer sa concentration en collagène qui était de 5 mg/ml environ.

### 1.2 Préparation d'un hydrogel de collagène I concentré

La solution de collagène à 5 mg/ml environ telle que préparée ci-dessus a été dissoute dans une solution d'acide acétique 0,5 M environ. La solution a été évaporée à l'air sous une hotte stérile jusqu'à atteindre une concentration finale en collagène de 40 mg/ml environ. L'évaporation du solvant a été contrôlée par pesée. La concentration finale de la solution a ensuite été confirmée par un dosage d'hydroxyproline.

La solution de collagène à 40 mg/ml environ ainsi obtenue a été introduite dans un moule, puis l'ensemble moule/solution de collagène a été centrifugé pour aplanir les irrégularités. Le moule résultant a ensuite été placé sous vapeurs d'ammoniaque pendant 12h environ, pour permettre la gélification et la fibrillogénèse du collagène I. L'hydrogel obtenu a été rincé plusieurs fois dans des bains de PBS stérile (i.e. qui a subi une stérilisation humide dans un autoclave) pour éliminer l'ammoniaque et ramener le pH à 7. Le pH a été contrôlé avant chaque lavage. L'hydrogel de collagène pur obtenu est dénommé **M_{A-H}.**

### 1.3 Déshydratation d'un hydrogel de collagène I concentré

L'hydrogel de collagène I tel qu'obtenu ci-dessus a été déshydraté progressivement par incubation dans plusieurs bains mixes THF/eau successifs : un bain THF/eau ayant une concentration volumique en THF de 30%, un bain THF/eau ayant une concentration volumique en THF de 50%, un bain THF/eau ayant une concentration volumique en THF de 70%, un bain THF/eau ayant une concentration volumique en THF de 95% et enfin un bain de THF pur. L'incubation dans chacun des bains a duré 1h environ.

Suite à l'étape de déshydratation, on a obtenu un hydrogel déshydraté de collagène pur **M_{A-HD}**.

L'hydrogel déshydraté de collagène pur **M_{A-HD}** peut être rincé avec du PBS et lyophilisé pour donner un matériau sec de collagène pur **M_{A-S}** puis **M_{A-S}** peut être réhydraté avec du PBS pour reformer un hydrogel de collagène pur dénommé **M_{A-HR}**.

### 1.4 Préparation du biomatériau composite synthétique M₁

Une solution comprenant 160 mg/ml de PLGA 30-60 KDa et 10⁻² M (i.e. 4,16 mg/ml) de spironolactone a été préparée en dissolvant la spironolactone dans du THF, puis en dissolvant le PLGA dans le mélange précédent.

L'hydrogel déshydraté de collagène pur **M_{A-HD}** tel que préparé ci-dessus a été imprégné pendant 12h environ avec la solution comprenant le PLGA et la spironolactone, le volume de la solution étant au moins 5 fois plus grand que celui de l'hydrogel de collagène pur déshydraté.

Après l'imprégnation, l'hydrogel composite obtenu a été rincé 3 fois 30 secondes environ avec du THF pur pour éliminer l'excès de PLGA, puis 3 fois 30 minutes environ avec du PBS stérile pour figer le polymère au sein du réseau de collagène fibrillé et former le biomatériau composite de l'invention **M_{1-H}** sous la forme d'un hydrogel composite.

Une forme sèche dudit biomatériau composite a également été obtenue. Pour ce faire, le biomatériau composite **M_{1-H}** a été plongé dans de l'azote liquide pendant 10 min environ pour éviter la formation de glace cristalline, puis lyophilisé pendant 24h environ (température inférieure à -40°C, vide à 100 µBarr), pour former le biomatériau composite de l'invention **M_{1-S}** sous la forme d'un matériau composite sec.

Le matériau composite sec a ensuite été réhydraté par ajout d'un tampon phosphate salin pour former un matériau **M_{1-HR}** sous la forme d'un hydrogel composite.

Le rapport massique collagène/polymère organique dans ledit matériau biocomposite était de 1/1.

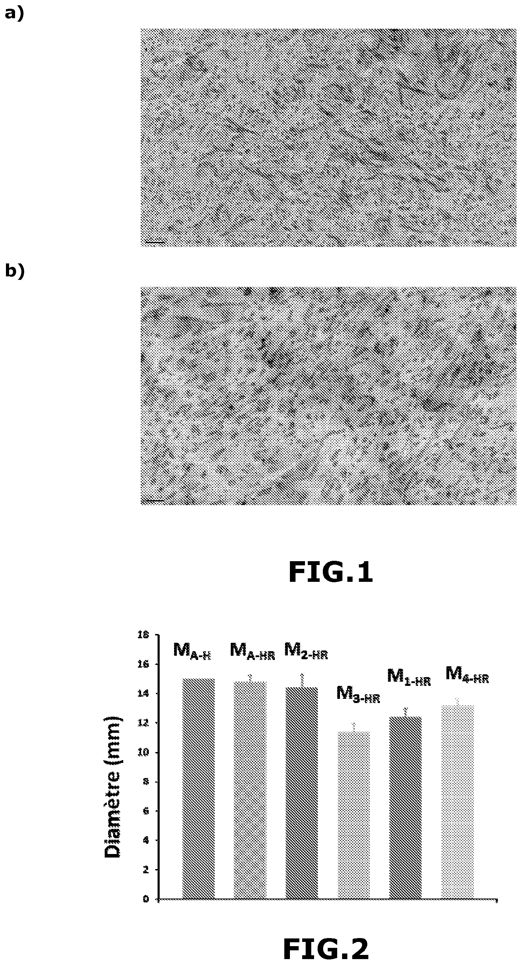

La figure 1 montre une image par microscopie électronique en transmission d'un hydrogel de collagène pur **M_{A-H}** tel qu'obtenu dans l'exemple 1.2 (figure la) et qui ne fait pas partie de l'invention, et une image du biomatériau composite **M_{1-HR}** sous la forme d'un hydrogel composite conforme à l'invention obtenu dans l'exemple 1.4 (figure 1b).

La figure 1 montre un biomatériau composite homogène dans lequel la structure fibrillaire et striée du collagène a été conservée. Par ailleurs, on ne distingue pas de domaines microscopiques de polymère PLGA, ce qui signifie que le polymère est uniformément réparti au sein du collagène.

Le tableau ci-dessous répertorie les valeurs des angles de contact des différents polymères organiques utilisés dans l'exemple 1 et les exemples ci-après :

| | PLGA | PLGA | PLGA | PCL |
|---|---|---|---|---|
| | 7-17 KDa | 24-38 KDa | 30-60 KDa | 14 KDa |
| Angle de contact (°) | 68,73 | 70,03 | 71,03 | 109,33 |
| Somme des écarts moyens (°) | 0,78 | 0,49 | 0,51 | 1,49 |

### Exemple 2 : préparation d'autres biomatériaux composites synthétiques conformes au premier objet de l'invention

Des biomatériaux composites **M_{2-HR}**, **M_{3-HR}** et **M_{4-HR}** ont été préparés en imprégnant l'hydrogel déshydraté de collagène pur **M_{A-HD}** tel qu'obtenu dans l'exemple 1.3 ci-dessus avec les solutions d'imprégnation respectives suivantes :
- une solution comprenant 10⁻² M environ de spironolactone et 160 mg/ml environ de PLGA 7-17 KDa,
- une solution comprenant 10⁻² M environ de spironolactone et 160 mg/ml environ de PLGA 24-38 KDa, et
- une solution comprenant 10⁻² M environ de spironolactone et 160 mg/ml environ de PCL 14 KDa.

Les étapes d'imprégnation, de rinçage, de lyophilisation et de réhydratation sont identiques à celles décrites dans l'exemple 1.4 ci-dessus.

La figure 2 représente le diamètre (en mm) des biomatériaux composites sous la forme d'hydrogels composites conformes à l'invention **M_{1-HR}**, **M_{2-HR}**, **M_{3-HR}** et **M_{4-HR}** et à titre comparatif le diamètre d'un hydrogel de collagène pur non conforme à l'invention **M_{A-H}** tel qu'obtenu dans l'exemple 1.2 et d'un hydrogel de collagène pur **M_{A-HR}** tel qu'obtenu dans l'exemple 1.3.

La figure 2 montre que la présence dans l'hydrogel d'un principe actif et d'un polymère organique hydrophobe tels que définis dans l'invention n'a que peu ou pas d'influence sur le diamètre de l'hydrogel obtenu. Les hydrogels composites ont donc une bonne capacité à s'hydrater, proche de celle du collagène pur et ne se rétractent pas.

La figure 3 représente la masse de polymère organique (en mg) présente dans les biomatériaux composites sous la forme de matériaux composites secs conformes à l'invention **M**_{**1**-**S**}, **M_{2-S}**, **M_{3-S}** et **M_{4-S}**.

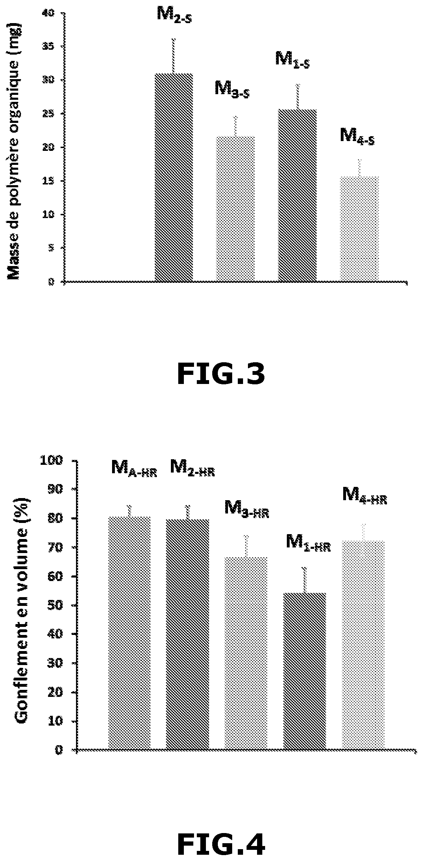

La figure 3 montre que le biomatériau composite de l'invention peut incorporer une quantité de polymère organique plus importante lorsque le PLGA 7-17 KDa est utilisé (**M_{2-S}**). Les capacités d'intégration des polymères organiques testés sont toutefois convenables et sont supérieures à 15 mg environ, et vont de préférence de 20 à 31 mg environ, pour une masse de collagène de l'ordre de 25 mg.

La figure 4 représente le gonflement en volume des biomatériaux composites sous la forme d'hydrogels composites conformes à l'invention **M_{1-HR}**, **M_{2-HR}**, **M_{3-HR}** et **M_{4-HR}** et à titre comparatif d'un hydrogel de collagène pur **M_{A-HR}** tel qu'obtenu dans l'exemple 1.3 (en % par rapport au volume initial de l'hydrogel de collagène pur **M_{A-H}**).

La figure 4 montre une bonne capacité de gonflement des hydrogels composites conformes à l'invention (55% à 80% environ), voire similaire à celle d'un hydrogel de collagène pur lorsque le PLGA 7-17 KDa est utilisé.

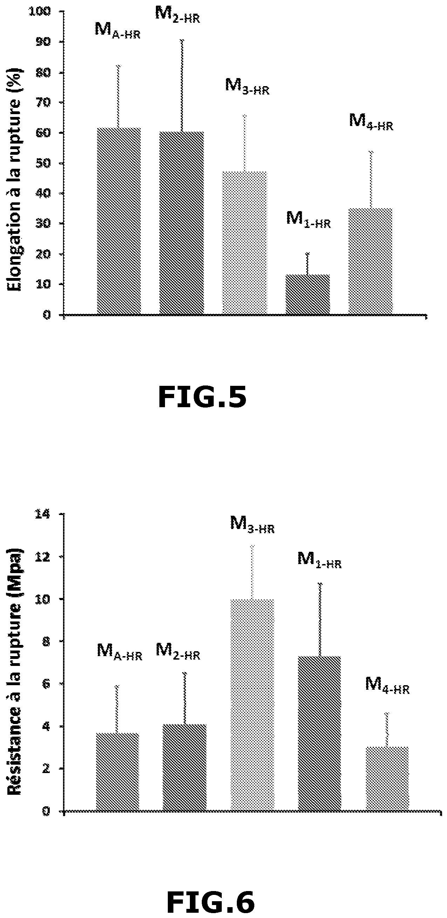

La figure 5 représente l'élongation à la rupture des biomatériaux composites sous la forme d'hydrogels composites conformes à l'invention **M_{1-HR}**, **M_{2-HR}**, **M_{3-HR}** et **M_{4-HR}**, et à titre comparatif celle d'un hydrogel de collagène pur **M_{A-HR}** tel qu'obtenu dans l'exemple 1.3 (en % de la longueur initiale de chacun des matériaux testés).

La figure 6 représente la résistance à la rupture (en MPa) des biomatériaux composites sous la forme d'hydrogels composites conformes à l'invention **M_{1-HR}**, **M_{2-HR}**, **M_{3-HR}** et **M_{4-HR}**, et à titre comparatif celle d'un hydrogel de collagène pur **M_{A-HR}** tel qu'obtenu dans l'exemple 1.3.

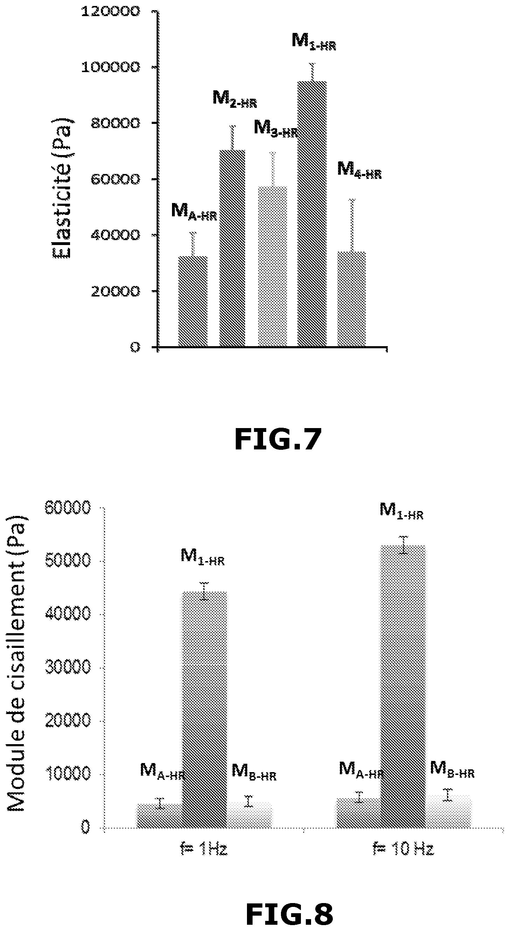

La figure 7 représente l'élasticité (en Pa) des biomatériaux composites (Module de Young) sous la forme d'hydrogels composites conformes à l'invention **M_{1-HR}**, **M_{2-HR}**, **M_{3-HR}** et **M_{4-HR}**, et à titre comparatif celle d'un hydrogel de collagène pur **M_{A-HR}** tel qu'obtenu dans l'exemple 1.3.

La figure 8 représente le module de cisaillement (en Pa) à différentes fréquences (1 Hz et 10 Hz environ) du biomatériau composite sous la forme d'un hydrogel composite conforme à l'invention **M_{1-HR}**, et à titre comparatif celui d'un hydrogel de collagène pur **M_{A-HR}** tel qu'obtenu dans l'exemple 1.3 et celui d'un hydrogel de collagène imprégné par de la spironolactone (i.e. sans polymère organique) **M_{B-HR}** obtenu à partir du matériau déshydraté de collagène pur **M_{A-HD}** tel qu'obtenu dans l'exemple 1.3 qui a été imprégné avec une solution comprenant 10⁻² M environ de spironolactone dans du THF et rincé, lyophilisé et réhydraté conformément à l'exemple 1.4 ci-dessus. On constate donc une nette amélioration de la tenue mécanique du biomatériau composite de l'invention par rapport à un hydrogène de collagène pur ou un hydrogène de collagène pur comprenant la spironolactone.

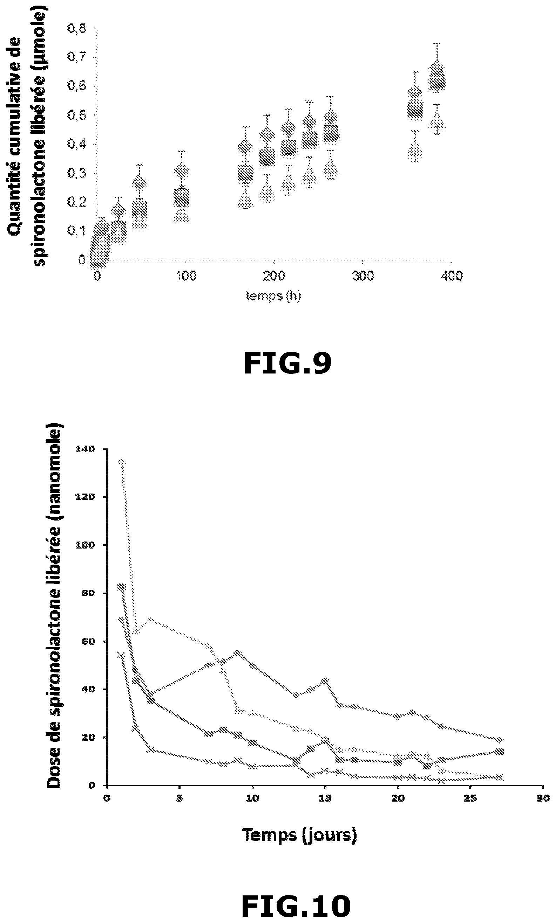

La figure 9 représente le profil de libération de la spironolactone des biomatériaux composites sous la forme de matériaux composites secs conformes à l'invention **M_{1-S}** (courbe avec les triangles), **M_{1-S1}** (courbe avec les losanges) et **M_{1-S2}** (courbe avec les carrés). Les différentes courbes montrent la quantité cumulative de spironolactone libérée (en nmole) en fonction du temps (en heures).

Il convient de noter que les biomatériaux initialement utilisés dans cette expérience sont sous la forme de matériaux composites secs. Toutefois, ils sont réhydratés instantanément lors de la mesure du suivi de libération du principe actif. Il en est de même pour les mesures de la toxicité des biomatériaux et de l'activité biologique de la spironolactone telles que décrites ci-après.

Les biomatériaux composites **M_{1-S1}** et **M_{1-S2}** ont été préparés comme dans l'exemple 1, sauf en ce qui concerne la concentration en PLGA dans la solution d'imprégnation qui était de 40 mg/ml environ pour **M_{1-S1}** et de 80 mg/ml environ pour **M_{1-S2}** (au lieu de 160 mg/ml environ pour **M_{1-S}**).

On constate qu'une concentration de 160 mg/ml environ est préférée pour favoriser la libération d'une dose constante de spironolactone en fonction du temps et obtenir un meilleur contrôle de la libération de la spironolactone. Il convient de noter que les trois biomatériaux composites conformes à l'invention n'ont libéré que de 30 à 60% environ de spironolactone en 400h (environ 2 semaines).

La figure 10 représente le profil de libération de la spironolactone des biomatériaux composites de l'invention **M_{1-S}** (courbe avec les croix), **M_{2-S}** (courbe avec les losanges), **M_{3-S}** (courbe avec les carrés) et **M_{4-S}** (courbe avec les triangles). Les différentes courbes montrent la dose quotidienne de spironolactone libérée (en nanomole par jour) en fonction du temps (en jours).

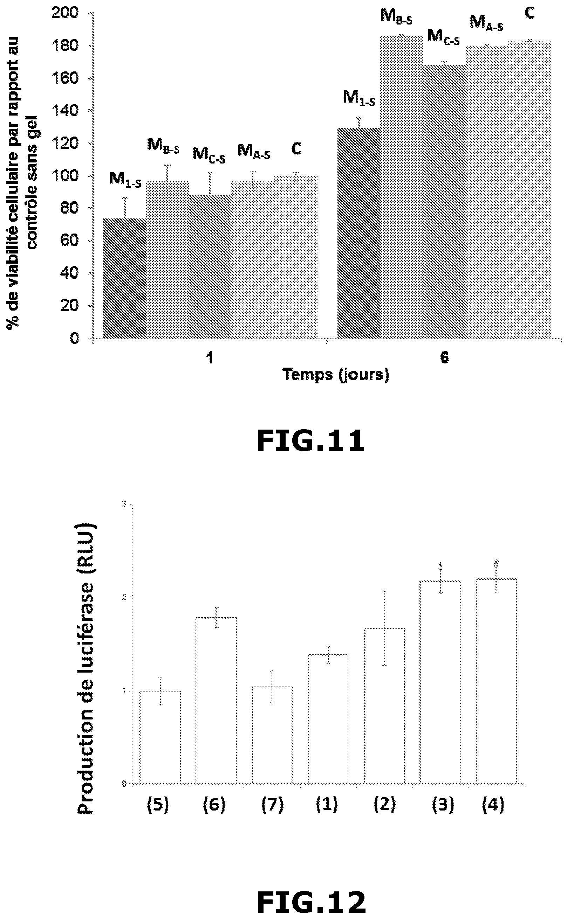

La figure 11 représente la toxicité du biomatériau composite de l'invention **M_{1-S}** sur des cellules (fibroblastes) et à titre comparatif :
- celle d'un matériau sec de collagène imprégné de spironolactone **M_{B-S}** obtenu à partir du matériau déshydraté de collagène pur **M_{A-HD}** tel qu'obtenu dans l'exemple 1.3 qui a été imprégné avec une solution comprenant 10⁻² M environ de spironolactone dans du THF, rincé et lyophilisé conformément à l'exemple 1.4 ci-dessus,
- celle d'un matériau sec de collagène imprégné de PLGA **M_{C-S}** obtenu à partir du matériau déshydraté de collagène pur **M_{A-HD}** tel qu'obtenu dans l'exemple 1.3 qui a été imprégné avec une solution comprenant 160 mg/ml environ de PLGA 30-60 KDa dans du THF, rincé et lyophilisé conformément à l'exemple 1.4, et
- celle d'un matériau sec de collagène pur **M_{A-S}** tel qu'obtenu dans l'exemple 1.3.

Par ailleurs, C représente un contrôle négatif (puits contenant uniquement les cellules sans hydrogel). Les différents diagrammes montrent la viabilité cellulaire des matériaux **M**_{**1**-**S**}, **M_{B-S}**, **M_{C-S}** et **M_{A-S}** tels que définis ci-dessus (en %) à 1 jour et à 6 jours, par rapport au contrôle négatif C.

Au sixième jour les points sont tous au-dessus de 100 % car les cellules ont proliféré et l'étude est cumulative. On remarque que la spironolactone a un léger effet toxique sur les cellules puisque les deux triangles sont légèrement en-dessous du contrôle (ronds). En effet, au premier jour seulement 74 % des cellules ont survécu. Toutefois, cela n'empêche pas la prolifération cellulaire (les droites sont croissantes). La figure 11 montre également la non-toxicité liée au polymère organique hydrophobe PLGA puisque les carrés sont au même niveau que le contrôle (ronds).

La figure 12 représente l'activité biologique de la spironolactone libérée à partir d'un hydrogel après 15 jours d'incubation dans le PBS (avec changement du tampon tous les jours) ; lorsque l'on utilise :
(1) un mélange d'un biomatériau composite conforme à l'invention **M_{1-S}** avec de l'aldostérone à 10⁻⁸ M environ, et à titre comparatif :
(2) un mélange d'un matériau **M_{B-S}** avec de l'aldostérone à 10⁻⁸ M environ,
(3) un mélange d'un matériau **M_{C-S}** avec de l'aldostérone à 10⁻⁸ M environ,
(4) un mélange d'un matériau **M_{A-S}** avec de l'aldostérone à 10⁻⁸ M environ,
(5) un contrôle négatif (puits contenant le milieu de culture sans aldostérone),
(6) de l'aldostérone à 10⁻⁸ M environ, et
(7) un mélange d'aldostérone à 10⁻⁸ M environ et de la spironolactone à 10⁻⁶ M environ.

La figure 12 montre la production de la luciférase (en unité relative de lumière ou RLU) selon le type de milieu utilisé. On constate que l'activité de la luciférase est plus importante dans les conditions (6) que dans les conditions (5), ce qui signifie que l'aldostérone s'est bien fixée au récepteur minéralocorticoïde et a engendré une transcription forte. Lorsque l'on ajoute de la spironolactone à 10⁻⁶ M environ (conditions (7)), l'effet de l'aldostérone est inhibé, il n'y a pas d'activation du récepteur. Les conditions (1) utilisant le biomatériau composite **M_{1-S}** conforme à l'invention montrent une activité comparable à celle obtenue dans les conditions (7), ce qui indique que la spironolactone a bien été relarguée par le biomatériau composite **M_{1-S}**, tout en conservant son activité. Lorsqu'il n'y a pas de PLGA (conditions (2)), le collagène ne retient pas la spironolactone et la quantité restante de spironolactone est inactive ou peu concentrée pour agir contre l'aldostérone. Enfin, les deux dernières conditions (3) et (4) correspondent à des contrôles sans spironolactone et sont à la même hauteur que celle associée aux conditions (6) contenant uniquement de l'aldostérone à 10⁻⁸ M ; il n'y a donc pas d'effet de l'hydrogel de collagène, ni de l'hydrogel de collagène comprenant du PLGA.

### Exemple 3 : préparation d'autres biomatériaux composites synthétiques conformes au premier objet de l'invention

Des biomatériaux composites **M_{5-HR}** et **M_{6-HR}** ont été préparés en imprégnant l'hydrogel déshydraté de collagène pur **M_{A-HD}** tel qu'obtenu dans l'exemple 1.3 ci-dessus avec les solutions d'imprégnation respectives suivantes :
- une solution comprenant 10⁻² M environ de spironolactone et 160 mg/ml environ de PLA 10 KDa, et
- une solution comprenant 10⁻² M environ de spironolactone et 160 mg/ml environ de PLA 30 KDa.

Les étapes d'imprégnation, de rinçage, de lyophilisation et de réhydratation sont identiques à celles décrites dans l'exemple 1.4 ci-dessus.

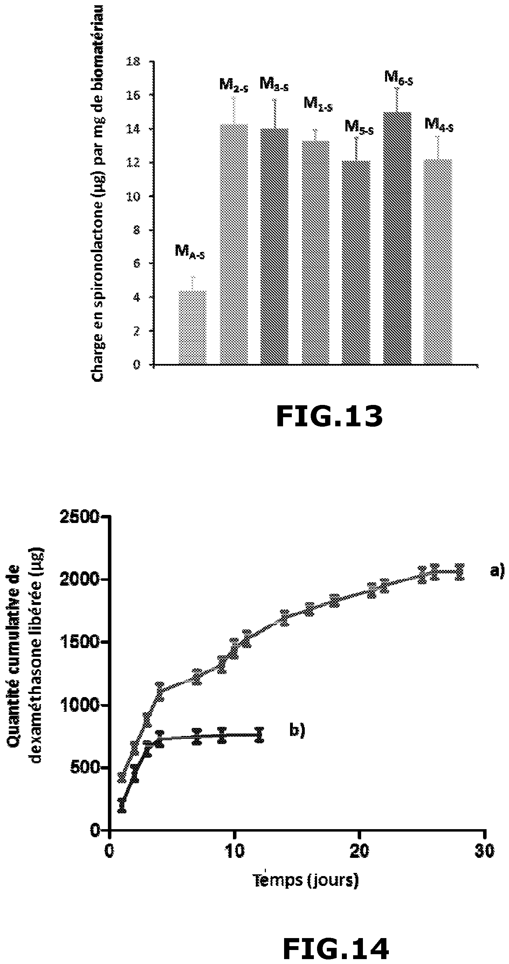

La figure 13 montre la charge en spironolactone (en µg) des biomatériaux composites sous la forme de matériaux composites secs conformes à l'invention **M**_{**1**-**S**}, **M_{2-S}**, **M_{3-S}**, **M_{4-S}**, **M_{5-S}** et **M_{6-S}**, par mg de biomatériau composite, et à titre comparatif la charge en spironolactone (en µg) du matériau sec de collagène pur **M_{A-S}** par mg dudit matériau.

D'autres biomatériaux composites **M_{7-HR}** et **M_{8-HR}** ont été préparés en imprégnant l'hydrogel déshydraté de collagène pur **M_{A-HD}** tel qu'obtenu dans l'exemple 1.3 ci-dessus avec les solutions d'imprégnation respectives suivantes :
- une solution comprenant 5,3x10⁻² M environ (i.e. 21 mg/ml) de dexaméthasone et 160 mg/ml environ de PLGA 7-17 KDa, et
- une solution comprenant 3,2x10⁻² M environ (i.e. 12,6 mg/ml) de dexaméthasone et 160 mg/ml environ de PLGA 7-17 KDa.

Les étapes d'imprégnation, de rinçage, de lyophilisation et de réhydratation sont identiques à celles décrites dans l'exemple 1.4 ci-dessus.

La figure 14 montre le profil de libération de la dexaméthasone des biomatériaux composites sous la forme de matériaux composites secs conformes à l'invention **M_{7-S}** (figure 14a)) et **M_{8-S}** (figure 14b)). Les différentes courbes montrent la quantité cumulative de dexaméthasone libérée (en µg) en fonction du temps (en jours).

## Revendications

1. Biomatériau composite synthétique comprenant du collagène, au moins un polymère organique et au moins un principe actif, **caractérisé en ce que** :
- le polymère organique est biodégradable, biocompatible, hydrophobe et il présente une température de transition vitreuse inférieure ou égale à 50°C et une masse molaire moyenne allant de 5 à 120 KDa,
- le collagène est sous la forme de fibrilles striées dans lesquelles la périodicité des striations est de 67 nm,
- le rapport massique collagène/polymère organique va de 10/1 à 1/3,
- le principe actif est un principe actif hydrophobe choisi parmi les anti-inflammatoires, les antibiotiques, les composés favorisant la réparation tissulaire ou la cicatrisation et un de leur mélange.

2. Biomatériau selon la revendication 1, **caractérisé en ce qu'**il est sous la forme d'un hydrogel composite et comprend de 70 à 95% en masse d'eau, par rapport à la masse totale du biomatériau composite.

3. Biomatériau selon la revendication 2, **caractérisé en ce qu'**il présente une élasticité allant de 50000 Pa à 100000 Pa.

4. Biomatériau selon la revendication 2 ou 3, **caractérisé en ce qu'**il présente une élongation à la rupture allant de 40 à 75%.

5. Biomatériau selon la revendication 1, **caractérisé en ce qu'**il est sous la forme d'un matériau composite sec et comprend au plus 10% en masse d'eau, par rapport à la masse totale du biomatériau composite.

6. Biomatériau selon l'une quelconque des revendications précédentes, **caractérisé en ce que** le polymère organique est choisi parmi les polyesters aliphatiques, les polyéthylènes glycols, les polyanhydrides et les poly(ortho-esters).

7. Biomatériau selon l'une quelconque des revendications précédentes, **caractérisé en ce que** le polymère organique est un polyester aliphatique choisi parmi un polyglycolide, un polylactide, un copolymère de glycolide et de lactide, un polylactone, et un polyhydroxyalcanoate.

8. Biomatériau selon l'une quelconque des revendications précédentes, **caractérisé en ce que** le polymère organique est sous la forme de nanodomaines présentant une taille moyenne inférieure ou égale à 700 nm.

9. Procédé de préparation d'un biomatériau composite tel que défini dans l'une quelconque des revendications 1 à 8, **caractérisé en ce qu'**il comprend au moins les étapes suivantes :
i) la préparation d'un hydrogel de collagène sous la forme de fibrilles striées dans lesquelles la périodicité des striations est de 67 nm, la concentration en collagène dans l'hydrogel étant d'au moins 10 mg/ml,
ii) la déshydratation de l'hydrogel tel que préparé à l'étape i) par incubation dudit hydrogel dans plusieurs solutions mixtes successives [solvant organique/solvant aqueux] présentant une proportion croissante en solvant organique, lesdits solvants aqueux et organique étant miscibles, suivi d'une dernière incubation dans une solution pure dudit solvant organique,
iii) la mise en contact de l'hydrogel déshydraté de l'étape ii) avec une solution d'imprégnation comprenant au moins un polymère organique et au moins un principe actif tels que définis à l'une quelconque des revendications 1 à 8, le rapport volumique : volume de la solution d'imprégnation/volume de l'hydrogel déshydraté étant supérieur ou égal à 3,
iv) le rinçage de l'hydrogel imprégné de l'étape iii) avec un solvant organique, puis avec un solvant aqueux.

10. Procédé selon la revendication 9, **caractérisé en ce que** l'étape i) est effectuée selon les sous-étapes suivantes :
i-1) la préparation d'une solution de collagène acido-soluble dont la teneur en collagène varie de 1 à 5 mg/ml,
i-2) l'évaporation à l'air de la solution de l'étape i-1), et
i-3) la mise en contact de la solution de l'étape i-2) avec une base.

11. Procédé selon la revendication 9 ou 10, **caractérisé en ce que** l'étape ii) est conduite en incubant l'hydrogel de l'étape i) dans une solution mixte solvant organique/solvant aqueux dont la teneur en solvant organique est de 20 à 30% en volume, puis dans une solution mixte solvant organique/solvant aqueux dont la teneur en solvant organique est de 40 à 50% en volume, puis dans une solution mixte solvant organique/solvant aqueux dont la teneur en solvant organique est de 60 à 70% en volume, puis dans une solution mixte solvant organique/solvant aqueux dont la teneur en solvant organique est de 80 à 95% en volume, puis dans une solution pure dudit solvant organique.

12. Procédé selon l'une quelconque des revendications 9 à 11, **caractérisé en ce que** l'incubation de l'étape ii) dans chacune des solutions dure de 30 min à 2h.

13. Procédé selon l'une quelconque des revendications 9 à 12, **caractérisé en ce que** la concentration en polymère organique de la solution d'imprégnation va de 20 à 500 mg/ml.

14. Procédé selon l'une quelconque des revendications 9 à 13, **caractérisé en ce qu'**il comprend en outre une étape v) de lyophilisation du biomatériau composite de l'étape iv).

15. Biomatériau composite tel que défini dans l'une quelconque des revendications 1 à 8, pour son usage médical.

16. Biomatériau composite tel que défini dans l'une quelconque des revendications 1 à 8, pour son utilisation dans le traitement de plaies chroniques.

17. Biomatériau composite tel que défini dans l'une quelconque des revendications 1 à 8, pour son utilisation dans le traitement préventif des infections après une chirurgie cardiaque ou colorectale.

18. Biomatériau composite tel que défini dans l'une quelconque des revendications 1 à 8, pour son utilisation dans le traitement des hernies de la paroi abdominale ou des éventrations.

19. Pansement thérapeutique comprenant une couche interne et une couche externe constituée d'un pansement secondaire choisi parmi un adhésif, une compresse, un bandage et un de leurs mélanges, **caractérisé en ce que** la couche interne comprend un biomatériau composite tel que défini dans l'une quelconque des revendications 1 à 8.

20. Renfort de paroi abdominale, **caractérisé en ce qu'**il comprend un biomatériau composite tel que défini dans l'une quelconque des revendications 1 à 8.

## Patentansprüche

1. Synthetisches zusammengesetztes Biomaterial, umfassend Kollagen, mindestens ein organisches Polymer und mindestens einen Wirkstoff, **dadurch gekennzeichnet, dass**
- das organische Polymer biologisch abbaubar, biokompatibel, hydrophob ist und eine Glaswandlungstemperatur aufweist, die weniger als oder gleich 50 °C beträgt, und eine mittlere Molarmasse, die von 5 bis 120 KDa reicht,
- das Kollagen die Form von geriffelten Fibrillen aufweist, in denen die Periodizität der Riffelungen 67 nm ist,
- das Massenverhältnis Kollagen/organisches Polymer von 10/1 bis 1/3 reicht,
- der Wirkstoff ein hydrophober Wirkstoff ist, ausgewählt aus Entzündungshemmern, Antibiotika, Verbindungen, die die Gewebereparatur oder die Narbenbildung begünstigen, oder einer ihrer Mischungen.

2. Biomaterial nach Anspruch 1, **dadurch gekennzeichnet, dass** es die Form eines zusammengesetzten Hydrogels aufweist und von 70 bis 95 Massenprozent Wasser mit Bezug auf die gesamte Masse des zusammengesetzten Biomaterials umfasst.

3. Biomaterial nach Anspruch 2, **dadurch gekennzeichnet, dass** es eine Elastizität aufweist, die von 50 000 Pa bis 100 000 Pa reicht.

4. Biomaterial nach Anspruch 2 oder 3, **dadurch gekennzeichnet, dass** es eine Bruchdehnung aufweist, die von 40 bis 75 % reicht.

5. Biomaterial nach Anspruch 1, **dadurch gekennzeichnet, dass** es die Form eines trockenen zusammengesetzten Materials aufweist und höchstens 10 Massenprozent Wasser mit Bezug auf die gesamte Masse des zusammengesetzten Biomaterials umfasst.

6. Biomaterial nach einem der vorhergehenden Ansprüche, **dadurch gekennzeichnet, dass** das organische Polymer ausgewählt ist aus aliphatischen Polyestern, Polyethylenglykolen, Polyanhydriden und Poly(ortho-estern).

7. Biomaterial nach einem der vorhergehenden Ansprüche, **dadurch gekennzeichnet, dass** das organische Polymer ein aliphatisches Polyester ist, ausgewählt aus Polyglycolid, Polylactid, Glycolid- und Lactidcopolymer, Polylacton und Polyhydroxyalcanoat.

8. Biomaterial nach einem der vorhergehenden Ansprüche, **dadurch gekennzeichnet, dass** das organische Polymer die Form von Nanodomänen aufweist, aufweisend eine mittlere Größe von weniger oder gleich 700 nm.

9. Verfahren zur Herstellung eines zusammengesetzten Biomaterials, wie in einem der Ansprüche 1 bis 8 definiert, **dadurch gekennzeichnet, dass** es mindestens die folgenden Schritte umfasst:
i) Herstellen eines Kollagenhydrogels in Form von geriffelten Fibrillen, in denen die Periodizität der Riffelung 67 nm ist, wobei die Konzentration von Kollagen im Hydrogel weniger als 10 mg/ml beträgt,
ii) Dehydrieren des Hydrogels, wie in Schritt i) hergestellt, durch Inkubation des Hydrogels in mehreren aufeinander folgenden gemischten Lösungen [organisches Lösemittel/wässriges Lösemittel], die eine zunehmende Proportion von organischem Lösemittel aufweisen, wobei die wässrigen und organischen Lösemittel mischbar sind, gefolgt von einer letzten Inkubation in einer reinen Lösung des organischen Lösemittels,
iii) In-Kontakt-Bringen des Hydrogels, dehydriert in Schritt ii), mit einer Imprägnierlösung, umfassend mindestens ein organisches Polymer und mindestens einen Wirkstoff, wie in einem der Ansprüche 1 bis 8 definiert, wobei das Volumenverhältnis: Volumen der Imprägnierlösung/Volumen des dehydrierten Hydrogels größer oder gleich 3 ist,
iv) Spülen des imprägnierten Hydrogels von Schritt iii) mit einem organischen Lösemittel, dann mit einem wässrigen Lösemittel.

10. Verfahren nach Anspruch 9, **dadurch gekennzeichnet, dass** Schritt i) gemäß den folgenden Unterschritten durchgeführt wird:
i-1) Herstellen einer säurelöslichen Kollagenlösung, deren Kollagengehalt von 1 bis 5 mg/ml variiert,
i-2) Verdampfen der Lösung von Schritt i-1) an der Luft, und
i-3) In-Kontakt-Bringen der Lösung von Schritt i-2) mit einer Base.

11. Verfahren nach Anspruch 9 oder 10, **dadurch gekennzeichnet, dass** Schritt ii) durchgeführt wird, indem das Hydrogel von Schritt i) in einer gemischten Lösung aus organischem Lösemittel/wässrigem Lösemittel inkubiert wird, deren Gehalt an organischem Lösemittel von 20 bis 30 Volumenprozent ist, dann in einer gemischten Lösung aus organischem Lösemittel/wässrigem Lösemittel, deren Gehalt an organischem Lösemittel von 40 bis 50 Volumenprozent ist, dann in einer gemischten Lösung aus organischem Lösemittel/wässrigem Lösemittel, deren Gehalt an organischem Lösemittel von 60 bis 70 Volumenprozent ist, dann in einer gemischten Lösung aus organischem Lösemittel/wässrigem Lösemittel, deren Gehalt an organischem Lösemittel von 80 bis 95 Volumenprozent ist, dann in einer reinen Lösung des organischen Lösemittels.

12. Verfahren nach einem der Ansprüche 9 bis 11, **dadurch gekennzeichnet, dass** die Inkubation von Schritt ii) in jeder der Lösungen von 30 min bis 2 h dauert.

13. Verfahren nach einem der Ansprüche 9 bis 12, **dadurch gekennzeichnet, dass** die Konzentration von organischem Polymer der Imprägnierlösung von 20 bis 500 mg/ml reicht.

14. Verfahren nach einem der Ansprüche 9 bis 13, **dadurch gekennzeichnet, dass** es außerdem einen Schritt v) des Lyophilisierens des zusammengesetzten Biomaterials von Schritt iv) umfasst.

15. Zusammengesetztes Biomaterial, wie in einem der Ansprüche 1 bis 8 definiert, für seine medizinische Verwendung.

16. Zusammengesetztes Biomaterial, wie in einem der Ansprüche 1 bis 8 definiert, für seine Verwendung bei der Behandlung von chronischen Wunden.

17. Zusammengesetztes Biomaterial, wie in einem der Ansprüche 1 bis 8 definiert, für seine Verwendung bei der präventiven Behandlung von Infektionen nach einer Herz- oder kolorektalen Chirurgie.

18. Zusammengesetztes Biomaterial, wie in einem der Ansprüche 1 bis 8 definiert, für seine Verwendung bei der Behandlung von Hernien der Bauchdecke oder der Darmdurchbrüche.

19. Therapeutischer Verband, umfassend eine innere Schicht und eine äußere Schicht, die aus einem sekundären Verband besteht, ausgewählt aus einem Kleber, einer Kompresse, einer Bandage und einer ihrer Mischungen, **dadurch gekennzeichnet, dass** die innere Schicht ein zusammengesetztes Biomaterial umfasst, wie in einem der Ansprüche 1 bis 8 definiert.

20. Verstärkung der Bauchwand, **dadurch gekennzeichnet, dass** sie ein zusammengesetztes Biomaterial umfasst, wie in einem der Ansprüche 1 bis 8 definiert.

## Claims

1. Synthetic composite biomaterial comprising collagen, at least one organic polymer and at least one active ingredient, **characterized in that**:
- the organic polymer is biodegradable, biocompatible, hydrophobic and has a glass transition temperature of less than or equal to 50°C, and an average molar mass ranging from 5 to 120 KDa,
- the collagen is in the form of striated fibrils in which the periodicity of the striations is 67 nm,
- the mass ratio of collagen/organic polymer ranges from 10/1 to 1/3,
- the active principle is a hydrophobic active ingredient chosen from among anti-inflammatories, antibiotics, compounds that promote tissue repair or scarring and one of their mixtures.

2. Biomaterial according to claim 1, **characterized in that** it is in the form of a composite hydrogel, and comprises from 70 to 95% by weight of water, based on the total weight of the composite biomaterial.

3. Biomaterial according to claim 2, **characterized in that** it has an elasticity ranging from 50000 Pa to 100000 Pa.

4. Biomaterial according to claim 2 or 3, **characterized in that** it has an elongation at break ranging from 40 to 75%.

5. Biomaterial according to claim 1, **characterized in that** it is in the form of a dry composite material and comprises at most 10% by weight of water, relative to the total weight of the composite biomaterial.

6. Biomaterial according to any one of the preceding claims, **characterized in that** the organic polymer is chosen from among aliphatic polyesters, polyethylene glycols, polyanhydrides and poly(ortho-esters).

7. Biomaterial according to any one of the preceding claims, **characterized in that** the organic polymer is an aliphatic polyester selected from a polyglycolide, a polylactide, a copolymer of glycolide and lactide, a polylactone, and a polyhydroxyalkanoate.

8. Biomaterial according to any one of the preceding claims, **characterized in that** the organic polymer is in the form of nanodomains having a mean size of less than or equal to 700 nm.

9. Method for the preparation of a composite biomaterial as defined in any one of claims 1 to 8, **characterized in that** it comprises at least the following steps:
i) preparation of a collagen hydrogel in the form of striated fibrils in which the periodicity of the striations is 67 nm, the concentration of collagen in the hydrogel being at least 10 mg/ml,
ii) dehydration of the hydrogel as prepared in step i) by incubating said hydrogel in several successive mixed solutions [organic solvent/aqueous solvent] having an increasing proportion of organic solvent, said aqueous and organic solvents being miscible, followed by a final incubation in a pure solution of said organic solvent,
iii) contacting the dehydrated hydrogel of step ii) with an impregnating solution comprising at least one organic polymer and at least one active ingredient as defined in any one of claims 1 to 8; the volume ratio: volume of the impregnating solution/volume of the dehydrated hydrogel being greater than or equal to 3,
iv) rinsing the impregnated hydrogel of step iii) with an organic solvent and then with an aqueous solvent.

10. Method according to claim 9, **characterized in that** step i) is carried out according to the following substeps:
i-1) preparation of an acid-soluble collagen solution whose collagen content varies from 1 to 5 mg/ml,
i-2) evaporation in air of the solution of step i-1), and
i-3) contacting the solution of step i-2) with a base.

11. Method according to claim 9 or 10, **characterized in that** step ii) is performed by incubating the hydrogel of step i) in a mixed organic solvent/aqueous solvent solution whose organic solvent content is 20 to 30% by volume, then in a mixed organic solvent/aqueous solvent solution whose content of organic solvent is 40 to 50% by volume, then in a mixed organic solvent/aqueous solvent solution whose organic solvent content is 60 to 70% by volume, then in a mixed organic solvent/aqueous solvent solution whose organic solvent content is 80 to 95% by volume, then in a pure solution of said organic solvent.

12. Method according to any one of claims 9 to 11, **characterized in that** the incubation of step ii) in each of the solutions lasts from 30 minutes to 2 hours.

13. Method according to any one of claims 9 to 12, **characterized in that** the organic polymer concentration of the impregnating solution is from 20 to 500 mg/ml.

14. Method according to any one of claims 9 to 13, **characterized in that** it further comprises a step v) lyophilization of the composite biomaterial of step iv).

15. Composite biomaterial as defined in any one of claims 1 to 8 for its medical use.

16. Composite biomaterial as defined in any one of claims 1 to 8 for its use in the treatment of chronic wounds.

17. Composite biomaterial as defined in any one of claims 1 to 8, for its use in the preventive treatment of infections after cardiac or colorectal surgery.

18. Composite biomaterial as defined in any one of claims 1 to 8 for use in the treatment of abdominal wall hernias or eventrations.

19. Therapeutic dressing comprising an inner layer and an outer layer consisting of a secondary dressing chosen from an adhesive, a compress, a bandage, and one of their mixtures, **characterized in that** the inner layer comprises a composite biomaterial as defined in any one of claims 1 to 8.

20. Abdominal wall reinforcement, **characterized in that** it comprises a composite biomaterial as defined in any one of claims 1 to 8.
